(19) **Europäisches Patentamt / European Patent Office / Office européen des brevets**

(11) **EP 1 237 000 B1**

(12) **FASCICULE DE BREVET EUROPEEN**

(45) Date de publication et mention
de la délivrance du brevet:
**14.04.2010 Bulletin 2010/15**

(51) Int Cl.:
*G01N 33/28* [(2006.01)]    *E21B 49/00* [(2006.01)]

(21) Numéro de dépôt: **02290415.5**

(22) Date de dépôt: **21.02.2002**

(54) **Méthode pour détecter et contrôler la formation d'hydrates en tout point d'une conduite où circulent des fluides pétroliers polyphasiques**

Verfahren zur Detektion und zur Kontrolle der Hydratbildung an allen Stellen von mehrphasigen Rohölflüssigkeiten transportierenden Rohren

Method for detecting and controlling hydrate formation at every point along a pipe in which polyphasic petroleum fluids flow

(84) Etats contractants désignés:
**DE GB IT NL**

(30) Priorité: **01.03.2001 FR 0102842**

(43) Date de publication de la demande:
**04.09.2002 Bulletin 2002/36**

(73) Titulaire: **Institut Français du Pétrole
92852 Rueil-Malmaison Cedex (FR)**

(72) Inventeurs:
- **Henriot, Véronique
  92500 Rueil-Malmaison (FR)**
- **Lachet, Véronique
  92360 Meudon La Forét (FR)**
- **Heintze, Eric
  92190 Meudon (FR)**

(56) Documents cités:
WO-A-96/32629    WO-A-98/45692
FR-A- 2 687 223    US-A- 6 028 992

- CALANGE S ET AL: "ONSET CRYSTALLIZATION TEMPERATURE AND DEPOSIT AMOUNT FOR WAXY CRUDES: EXPERIMENTAL DETREMINATION AND THERMODYNAMIC MODELLING" SPE INTERNATIONAL SYMPOSIUM ON OILFIELD CHEMISTRY, XX, XX, 18 février 1997 (1997-02-18), pages 283-290, XP000675714
- HENRIOT V ET AL: "TACITE: CONTRIBUTION OF FLUID COMPOSITION TRACKING ON TRANSIENT MULTIPHASE FLOW SIMULATION" PETROLE ET TECHNIQUES, ASSOCIATION FRANCAISE DES TECHNICIENS DU PETROLE. PARIS, FR, no. SUPPL 408, 1 mai 1997 (1997-05-01), pages OTC856301-10, XP000699908 ISSN: 0152-5425
- E. DURET ET AL: "PIPELINE BUNDLES MODEL IMPLEMENTED INTO A MULTIPHASE FLOW MODEL" SOCIETY OF PETROLEUM ENGINEERS INC. 62948, ANNUAL TECHNICAL CONFERENCE AND EXHIBITION DALLAS TEXAS, 1 - 4 octobre 2000, XP001050377
- DANESH A: "hydrate equilibrium data of methyl cyclopentane with methane or nitrogen" CHEMICAL ENGINEERING RESEARCH AND DESIGN, PART A, INSTITUTION OF CHEMICAL ENGINEERS, XX, vol. 72, 1 mars 1994 (1994-03-01), pages 197-200, XP002071473 ISSN: 0263-8762

EP 1 237 000 B1

## Description

La présente invention concerne une méthode pour détecter et contrôler la formation d'hydrates en tout point d'une conduite où circulent des fluides pétroliers polyphasiques.

## Etat de la technique

Produire des hydrocarbures depuis des gisements en eaux profondes (1500 - 3000 m) soulève un grand nombre de difficultés, essentiellement à cause des pressions élevées et des températures basses qui y règnent. Ces difficultés se rencontrent dans tous les domaines du savoir-faire pétrolier: le forage et les interventions sur puits, les installations de traitement (FPSO, FWHP, etc.) et leurs ancrages, les technologies sous-marines (pompage polyphasique, séparation sous-marine des phases des fluides pétroliers), les risers et ombilicaux ou le « flow assurance ».

Dans le domaine de la production proprement dit, l'exploitant est tenu de garantir le bon écoulement malgré de nombreux obstacles comme la formation de bouchons de fluide ou «slugging», les dépôts minéraux et organiques ou bien encore la formation d'hydrates. En offshore profond les pressions plus élevées et les températures plus basses favorisent la formation d'hydrates. Les interruptions de production durant lesquelles les fluides refroidissent accroissent encore les risques de formation d'hydrates.

Une détection tardive de la présence d'hydrates notamment par méconnaissance des conditions de leur formation dans les conduites, peut entraîner des problèmes de production coûteux : blocage complet des conduites de production et/ou de transport. Il est donc important pour l'exploitant, de disposer de moyens d'évaluation des risques de toute nature, de façon à mettre en place des techniques de régulation et d'intervention et ainsi de pouvoir garantir un bon écoulement des fluides en circulation.

Pour protéger et isoler thermiquement les conduites sous-marines, on les enferme dans un tube extérieur résistant à la pression hydrostatique (« souvent désigné par « bundles » par les gens de l'art). Le plus souvent, on regroupe plusieurs conduites pour former des faisceaux. Ces conduites regroupées ont souvent des fonctions différentes. Elles servent à la production d'hydrocarbures, à l'injection de fluides dans le gisement ou réservoir, à l'injection de gaz dans les conduites (« gas lift »), à la circulation de fluide de réchauffage, etc. Dans l'espace entre elles, on interpose par exemple un calorifugeage à faible conductivité thermique laissé à la pression atmosphérique ou mis sous vide, avec des cloisonnements placés à intervalles réguliers pour des raisons de sécurité.

Avec un outil efficace d'évaluation en continu des risques de formation d'hydrates et autres dépôts, l'exploitant peut intervenir soit par le biais d'un réchauffage des conduites soit par l'injection d'additifs. Cependant un tel outil est difficile à mettre en place et coûteux en temps de calcul surtout si on doit tenir compte de la composition détaillée des fluides pétroliers. Pour étudier plus facilement leur comportement, il est connu de les décrire comme une combinaison d'un nombre de composants ou pseudo composants beaucoup plus réduit que le nombre réel de constituants. On parle alors d'une composition regroupée par opposition à une composition détaillée.

Par les brevets FR 2 753 535 et FR 2 806 803 du demandeur, on connaît des méthodes pour prédire la température d'apparition de dépôts tels que des cires ou paraffines dans des bruts pétroliers comportant le regroupement ou « lumping » de tous leurs constituants en un nombre plus réduit de pseudo composants représentant chacun plusieurs classes d'hydrocarbures et dont les paramètres physico-chimiques sont déterminés par combinaison des paramètres correspondants d'un certain nombre d'hydrocarbures purs regroupés dans une base de données. Les pseudo composants de cette formulation regroupée, sont appliqués à un module thermodynamique permettant de déterminer différents paramètres indicatifs des conditions de formation des dépôts.

Différents logiciels de simulation existent sur le marché permettant de modéliser le comportement de fluides polyphasiques en circulation dans des pipelines.

Par les brevets ou demandes de brevet suivants : US 5 550 761, FR 2.756.044 (US 6 028 992) et FR 2 756 045 (US 5 960 187), FR 00/08 200 et FR 00/09 889 du demandeur, et aussi par les publications suivantes :

- Faille I. et Heintzé E., " A rough finite volume scheme for modeling two-phase flow in a pipeline", *Computers & Fluids* 28 (1999), et

- Pauchon C. et al, "TACITE: a comprehensive mechanistic model for two-phase flow", *6th BHRG Multiphase International Conference*, Cannes, France, June (1993),

on connaît notamment le code de simulation TACITE (marque déposée) qui simule le comportement transitoire des fluides polyphasiques en circulation en prenant en compte à tout instant leur composition. Avec un tel code prévisionnel, l'ingénieur de production est en mesure de définir les caractéristiques des réseaux d'acheminement de fluides, notamment d'hydrocarbures : conduites, séparateurs, vannes, systèmes de contrôle, etc. Un des objectifs importants dans ces opérations de simulation, c'est de prédire avec précision les caractéristiques des écoulements transitoires lors de situations telles que : variation du débit d'entrée, dépressurisation à la sortie, arrêts de production et redémarrage, nettoyage des conduites (« pigging »), apparition et propagation de bouchons liquides, formation de dépôts susceptibles de se former dans certaines conditions thermodynamiques, etc.

Les modes d'écoulement de fluides polyphasiques dans des tubes sont extrêmement variés et complexes. Les écoulements diphasiques, par exemple, peuvent être stratifiés, la phase liquide s'écoulant dans la partie inférieure de la conduite, ou intermittents avec une succession de bouchons liquides et gazeux, ou bien encore dispersés, le liquide étant entraîné sous forme de fines gouttelettes. Le mode d'écoulement et le glissement entre les phases varient notamment avec l'inclinaison des conduites par rapport à l'horizontale et dépendent du débit, de la température etc.

Le code TACITE, on le rappelle, est adapté à suivre les variations de la composition des mélanges d'hydrocarbures. Comme déjà décrit dans les brevets précités du demandeur, il y a une équation de conservation de masse pour chaque pseudo-composant. Il y a aussi une équation de quantité de mouvement et une équation pour l'énergie du mélange. Les équations correspondantes sont rappelées ci-après, après une définition des symboles et notations:

- $V$ vitesse absolue de phase ;
- $U$ vitesse superficielle ;
- $R$ fraction volumétrique par phase ;
- $\rho$ densité de phase ;
- $H$ enthalpie de phase ;
- $E$ énergie interne ;
- $P$ pression du mélange ;
- $T_w$ frottement pariétal ;
- $Q_w$ flux de chaleur pariétal entre le pipeline et le milieu environnant ;
- $\theta$ inclinaison du pipeline ;
- $g$ gravité ;
- $S$ section du pipeline ;
- $x$ fraction massique du composant dans une phase donnée ;
- $p$ nombre de phases (de 1 à 3) ;
- $N$ nombre de composants ;
- $k$ indice de phase ;
- $i$ indice de composant ;
- $m$ indice de mélange.
- L'équation d'équilibre de masse pour chaque composant est :

$$\frac{\partial}{\partial t}\left\{\sum_{k=1}^{p} S\left(\rho_k R_k x_i^k\right)\right\} + \frac{\partial}{\partial x}\left\{\sum_{k=1}^{p} S\left(\rho_k R_k x_i^k V_k\right)\right\} = 0 \qquad \left(i = 1, \cdots, N\right). \qquad (1)$$

- L'équation d'équilibre de la quantité de mouvement du mélange est :

$$\frac{\partial}{\partial t}\left\{\sum_{k=1}^{p} S\left(\rho_k R_k V_k\right)\right\} + \frac{\partial}{\partial x}\left\{\left(\sum_{k=1}^{p} S\left(\rho_k R_k V_k^2\right)\right) + P\right\} = S(T_w - \rho_m g \sin\theta). \qquad (2)$$

- L'équation d'équilibre d'énergie du mélange est :

$$\frac{\partial}{\partial t}\left\{\sum_{k=1}^{p} S\left(\rho_k E_k\right)\right\} + \frac{\partial}{\partial x}\left\{\sum_{k=1}^{p} S\left(\rho_k R_k V_k H_k\right)\right\} = S(Q_w - \rho_m g U_m \sin\theta). \qquad (3)$$

**Schéma numérique**

Le schéma numérique est conservatif et non dissipatif. Il fournit un bon bilan de masse et d'énergie en tout point de la conduite et à tout instant. Un schéma mixte implicite/explicite est utilisé pour optimiser le temps de calcul et la capacité de suivi des fronts des ondes de taux de vide, ce qui est particulièrement important dans le cas où la configuration du terrain suivi par le pipeline ou sa configuration propre, favorise la formation de bouchons de liquide avec propagation d'ondes de vide dans les deux directions opposées du pipeline, phénomènes que les spécialistes désignent couramment par "terrain slugging" ou "severe slugging".

### Module thermodynamique

Le code de simulation TACITE comporte un "flash" thermodynamique intégré i.e. un sous-programme intégré de calcul des propriétés thermodynamiques (équilibre liquide-vapeur, composition de chacune des phases) à l'aide d'une équation d'état. Ce flash réalise des calculs d'équilibre thermodynamique di et tri-phasiques pour des mélanges d'hydrocarbures incluant de l'eau. Les équations d'état cubiques de Peng-Robinson (1) et de Soave-Redlich-Kwong (2) sont utilisées pour modéliser les propriétés thermodynamiques à l'équilibre des phases. Ces équations sont définies dans les publications suivantes :

- D.-Y. Peng D.Y. et al, "A new two-constant equation of state", Ind. Eng. Chem. Fund. 15, 59 (1976);

- Soave G., "Equilibrium constants from a modified Redlich-Kwong equation of state", Chem. Eng. Sci. 27, 1197 (1972).

Dans les deux cas, les volumes moléculaires peuvent être corrigés par la méthode de Péneloux décrite dans la publication suivante :
Péneloux A. et al, "A consistent correction for Redlich-Kwong-Soave volumes", Fluid Phase Equilibria 8, 7 (1982).
Le module dit flash assure un suivi précis de la composition des fluides aussi bien dans l'espace que dans le temps, durant toute la simulation.
Ce suivi compositionnel rend le code TACITE particulièrement apte à prédire avec précision le risque de la formation d'hydrates, comme on le verra par la suite.

### Module hydrodynamique

Le module hydrodynamique calcule le régime d'écoulement, la vitesse de glissement entre phases et les termes de frottement.

### La méthode selon l'invention

La méthode selon l'invention permet de détecter en continu en tout point d'une conduite où circule un mélange polyphasique de fluides pétroliers, les conditions thermodynamiques de formation d'hydrates. Elle comporte l'utilisation d'un module hydrodynamique mécanistique et d'un module thermodynamique compositionnel intégré pour définir les propriétés des phases et la résolution d'équations de conservation de la masse, de conservation de la quantité de mouvement et de transfert d'énergie au sein du mélange, en considérant que le mélange de fluides est sensiblement à l'équilibre à chaque instant, que la composition du mélange polyphasique est variable tout le long de la conduite et que la masse de chaque constituant du mélange est définie globalement par une équation de conservation de la masse sans considération de son état de phase. Les fluides pétroliers sont regroupés en un nombre limité de pseudo-composants regroupant chacun certaines fractions d'hydrocarbures.
La méthode est caractérisée en ce que l'on détecte les conditions thermodynamiques de formation d'hydrates après avoir opéré un regroupement particulier des fluides pétroliers en pseudo-composants de façon à isoler les composants formateurs d'hydrates, avec définition pour chacun d'eux d'une fraction massique et d'un certain nombre de grandeurs physiques caractéristiques, et on applique aux modules thermodynamique et hydrodynamique, des données relatives à ces pseudo-composants sélectionnés de manière à déterminer en tout point la température (Td) de dissociation des hydrates.
L'invention concerne aussi une méthode pour contrôler en continu et en tout point d'une conduite où circulent des fluides pétroliers polyphasiques, la formation d'hydrates. Elle comporte la détection des conditions thermodynamiques de formation des hydrates comme défini ci-dessus, l'utilisation d'un organe de contrôle pour comparer la température des fluides pétroliers à cette température de dissociation (Td) et l'application commandée par cet organe de contrôle, de mesures destinées à combattre la formation des hydrates.
Suivant un premier mode de mise en oeuvre, on utilise des moyens de chauffage associés à la conduite pour élever la température des fluides pétroliers au-dessus de la température de dissociation. Dans le cas où la conduite est incluse avec au moins un deuxième conduit dans un tube isolé du milieu extérieur, on utilise le deuxième conduit pour y faire circuler un fluide chaud.

Suivant un deuxième mode de mise en oeuvre, sur commande de l'organe de contrôle (C), on injecte des inhibiteurs d'hydrates dans la conduite.

**Présentation sommaire des figures**

D'autres caractéristiques et avantages de la méthode selon l'invention, apparaîtront à la lecture de la description ci-après d'un exemple non limitatif de réalisation, en se référant aux dessins annexés où :

- la Fig.1 montre un organigramme comparé des opérations de simulation dans le cadre de l'application du code TACITE selon que l'on étudie ou non le risque de formation d'hydrates ;

- la Fig.2 montre un organigramme de déroulement des opérations de simulation qui se répètent en tous les points d'un maillage de pipeline ;

- la Fig.3 montre schématiquement un mode de contrôle que l'on peut appliquer pour mettre en oeuvre toute intervention visant à combattre la formation d'hydrates ;

- la Fig.4 montre la section transversale d'un tube le long duquel courent plusieurs ensembles de tubes ou lignes de circulation de fluides ;

- la Fig.5 montre une comparaison des températures de dissociation d'hydrates calculées aux températures expérimentales dans le cas du méthane. L'histogramme est établi à partir de 158 points expérimentaux ;

- la Fig.6 montre une comparaison des températures de dissociation d'hydrates calculées aux températures expérimentales dans le cas de l'éthane. L'histogramme est établi à partir de 126 points expérimentaux ;

- la Fig.7 montre une comparaison des températures de dissociation d'hydrates calculées aux températures expérimentales dans le cas du propane. L'histogramme est établi à partir de 93 points expérimentaux ;

- la Fig.8 montre une comparaison des températures de dissociation d'hydrates calculées aux températures expérimentales dans le cas de l'isobutane. L'histogramme est établi à partir de 43 points expérimentaux ;

- la Fig.9 montre une comparaison des températures de dissociation d'hydrates calculées aux températures expérimentales dans le cas du dioxyde de carbone. L'histogramme est établi à partir de 102 points expérimentaux ;

- la Fig.10 montre une comparaison des températures de dissociation d'hydrates calculées aux températures expérimentales dans le cas de l'azote. L'histogramme est établi à partir de 57 points expérimentaux ;

- la Fig.11 montre une comparaison des températures de dissociation d'hydrates calculées aux températures expérimentales dans le cas du sulfure d'hydrogène. L'histogramme est établi à partir de 64 points expérimentaux ;

- la Fig. 12 montre une comparaison des températures de dissociation d'hydrates calculées aux températures expérimentales dans le cas de mélanges d'hydrocarbures. L'histogramme est établi à partir de 55 points expérimentaux provenant de 8 mélanges différents ;

- la Fig.13 montre schématiquement un exemple de raccordement d'un pipeline avec un riser avec un point d'injection de gaz (pour gas lift) ;

- la Fig.14 montre dans un diagramme bidimensionnel longueur de conduite-temps, un exemple de domaine que l'on peut calculer par la méthode d'apparition d'hydrates dans l'agencement de la Fig.13 ;et

- la Fig.15 montre dans un diagramme bidimensionnel pression-température deux courbes locales d'apparition d'hydrates dans les conduites, selon que l'on tient compte ou non d'une modification de la composition du mélange en circulation.

**DESCRIPTION DETAILLEE**

**Présentation du module thermodynamique**

Structure des hydrates

Les hydrates sont des composés solides constitués de molécules d'hydrocarbures (méthane, éthane, dioxyde de carbone etc.) piégées à haute pression et basse température à l'intérieur de cages approximativement sphériques formées par un réseau cristallin tridimensionnel de molécules d'eau. Cette structure est métastable en l'absence d'hydrocarbures et nécessite un taux de remplissage minimal pour assurer sa stabilisation. A la différence du réseau cristallin de la glace, celui des hydrates peut demeurer stable au-dessus de 0˚C.

Des mesures par diffraction aux rayons X réalisées dans les années 50 ont permis de mettre en évidence deux structures cristallines des hydrates selon la nature des molécules d'insertion présentes. Dans les deux cas, il s'agit de structures cubiques organisées en réseaux de petites et grandes cavités. Le tableau ci-après montre l'occupation possible des différentes cavités selon la nature des molécules formatrices d'hydrates mises en jeu (+ : occupation possible de la cavité par la molécule ; - : taille de la cavité insuffisante pour contenir la molécule).

| | Structure I | | Structure II | |
|---|---|---|---|---|
| Molécules | Petite cavité | Grande cavité | Petite cavité | Grande cavité |
| $N_2$ | + | + | + | + |
| $CO_2$ | + | + | + | + |
| $H_2S$ | + | + | + | + |
| $C_1$ | + | + | + | + |
| $C_2$ | - | + | - | + |
| $C_3$ | - | - | - | + |
| $iC_4$ | - | - | - | + |
| $nC_4$ | - | - | - | + |

Le réseau de la structure I est de type cubique face centrée. Chaque maille contient 46 molécules d'eau organisées en :

- 2 petites cavités de 3,95 Å de rayon,
- 6 grandes cavités de 4,33 Å de rayon.

Le cristal de la structure II a le réseau du diamant. Une maille contient 136 molécules d'eau et 24 cavités :

- 16 petites cavités de rayon 3,91 Å,
- 8 grandes cavités de rayon 4,73 Å.

Une troisième structure, la structure H, a été également identifiée. Son réseau cristallin, de type hexagonal, est constitué de 34 molécules d'eau et de 6 cavités :

- 2 petites cavités de 3,91 Å de rayon,
- 3 cavités intermédiaires d'un rayon de 4,06 Å,
- 1 grande cavité de 5,71 Å de rayon.

Cette structure est beaucoup plus rare que les deux précédentes, elle se rencontre principalement dans le cas d'alcanes ou d'alcènes cycliques.

Thermodynamique de l'équilibre de phases en présence d'hydrates

Tout calcul de prédiction d'hydrates est basé sur le critère d'équilibre de l'eau en phase hydrate et en phase aqueuse, c'est-à-dire sur l'égalité du potentiel chimique de l'eau dans la phase hydrate et du potentiel chimique de l'eau dans la phase aqueuse :

$$\mu_{\text{eau}}^{\text{aqueuse}}\left(P,T\right) = \mu_{\text{eau}}^{\text{hydrate}}\left(P,T\right). \tag{1}$$

Selon la théorie statistique de Van Der Waals et Platteeuw décrite dans la publication suivante :Van der Waals, J.H., et al., "Clathrate Solutions", *Adv. Chem. Phys.,* 2, 1, (1959),
le potentiel chimique de l'eau dans la phase hydrate peut s'écrire :

$$\mu_{\text{eau}}^{\text{hydrate}}\left(P,T\right) = \mu_{\text{eau}}^{\text{hydrate-vide}}\left(P,T\right) + RT\sum_{i}\nu_{i}\,\ln\!\left(1 - \sum_{k}\theta_{ik}\right), \tag{2}$$

avec :

- $\mu_{\text{eau}}^{\text{hydrate-vide}}$ : potentiel chimique de l'eau dans le réseau cristallin en l'absence de molécule d'hydrocarbure,

- $\nu_i$ : rapport du nombre de cavités de type $i$ (petite ou grande) sur le nombre de molécules d'eau dans un monocristal,

- $\theta_{ik}$ : probabilité pour qu'une cavité de type $i$ soit occupée par une molécule hydrocarbonée de type $k$. D'après la théorie de l'adsorption de Langmuir, cette probabilité peut se calculer par :

$$\theta_{ik} = \frac{C_{ik}\,f_k}{1 + \sum_{j} C_{ij}\,f_j}, \tag{3}$$

où $f_k$ est la fugacité du composant $k$ et $C_{ik}$ est le coefficient d'adsorption de Langmuir d'une molécule $k$ dans une cavité de type $i$.

Les coefficients de Langmuir peuvent être exprimés à partir du potentiel d'interaction entre les molécules d'eau et les molécules de gaz incluses dans les cavités de la structure cristalline d'hydrate :

$$C_{ik} = \frac{4\pi}{kT}\int_0^R \exp\!\left(\frac{-W(r)}{kT}\right) r^2\,dr, \tag{4}$$

où k est la constante de Boltzmann et W($r$) l'énergie potentielle d'interaction entre les molécules d'eau formant la cavité et la molécule de gaz se trouvant à une distance $r$ du centre de la cavité. Il existe plusieurs formes de potentiel, les plus utilisées sont le potentiel de Lennard-Jones, le potentiel de Kihara et le potentiel à puits carré.
Le potentiel de Lennard-Jones entre deux molécules s'écrit :

$$W(r) = 4\varepsilon\left[\left(\frac{\sigma}{r}\right)^{12} - \left(\frac{\sigma}{r}\right)^{6}\right], \tag{5}$$

avec :

- $\varepsilon$: profondeur du puits,

- $r$ : distance entre les centres des molécules,
- $\sigma$ : diamètre d'exclusion, $r$ tel que $W(\sigma) = 0$.

Le potentiel de Kihara est identique à celui de Lennard-Jones, mais les molécules sont considérées comme des sphères de rayon non nul. Son expression analytique est la suivante :

$$W(r) = \infty \qquad\qquad\qquad \text{si } r \leq 2a \qquad (6)$$

$$W(r) = 4\varepsilon\left[\left(\frac{\sigma}{r-2a}\right)^{12} - \left(\frac{\sigma}{r-2a}\right)^{6}\right] \qquad \text{si } r > 2a$$

avec :

- $\varepsilon$ : profondeur du puits,
- $\sigma$ : tel que $W(\sigma+2a) = 0$,
- $a$ : rayon moyen des 2 molécules,
- $r$ : distance entre les centres des molécules.

Enfin, le potentiel à puits carré est donné par :

$$
\begin{aligned}
W(x) &= \infty \quad si \quad 0 \leq x < \sigma \\
W(x) &= -\varepsilon \quad si \quad \sigma \leq x \leq \alpha\sigma \\
W(x) &= 0 \quad si \quad x > \alpha\sigma
\end{aligned}
\qquad (7)
$$

Les différents paramètres figurant dans les expressions de ces potentiels sont ajustés sur des données expérimentales d'équilibre d'hydrates.

Le potentiel chimique de l'eau dans la phase aqueuse s'écrit :

$$\mu_{\text{eau}}^{\text{aqueuse}}\left(P,T\right) = \mu_{\text{eau}}^{0}\left(P,T\right) + RT\ln\left(\gamma x\right), \qquad (8)$$

où $\gamma$ est le coefficient d'activité de l'eau, x la fraction molaire de l'eau dans la phase aqueuse et $\mu_{\text{eau}}^{0}\left(P,T\right)$ le potentiel chimique de l'eau pure. C'est dans ce calcul de l'activité de l'eau en phase aqueuse que sont entre autres pris en compte les effets des sels et des alcools.

En égalisant les équations (2) et (8), la condition d'équilibre recherchée (1) s'écrit :

$$\mu_{\text{eau}}^{0}\left(P,T\right) - \mu_{\text{eau}}^{\text{hydrate-vide}}\left(P,T\right) = RT\sum_{i}\nu_{i}\,\ln\left(1 - \sum_{k}\theta_{ik}\right) - RT\ln\left(\gamma x\right). \qquad (9)$$

En fixant la pression, cette équation nous permet d'accéder à la température d'équilibre ou inversement, en fixant la température nous obtenons la pression d'équilibre.

Les différents modules de prédiction d'hydrates sont tous basés sur le formalisme décrit ci-dessus. Ces modules vont essentiellement se différencier dans le choix du potentiel d'interaction eau - hydrocarbure, dans le choix de l'équation d'état utilisée pour le calcul des fugacités ainsi que dans le module de calcul de l'activité de l'eau en phase liquide. Dans ce qui suit, nous présentons plus précisément le module que nous avons adapté pour les besoins de TACITE.

Module implémenté dans TACITE

Le module de prédiction d'hydrates implémenté dans TACITE s'inspire du module de Munck tel que décrit par Munck, J. et al, "Computations of the formation of gas hydrates", Chem. Eng. Sci 43, 2661 (1988). Il permet de calculer la température de dissociation des hydrates à pression donnée dans le cas d'un gaz, d'un liquide ou d'un mélange gaz - liquide diphasique en contact avec de l'eau liquide.

Constantes de Langmuir :

Ce module utilise un potentiel *W(r)* de type puits carré, ce qui conduit à une expression de la constante de Langmuir du composé *k* dans une cavité de type *i* sous la forme :

$$C_{ik} = \frac{A_{ik}}{T} \exp(\frac{B_{ik}}{T}) . \qquad (10)$$

Les valeurs des paramètres *A* et *B* des différents composés formant des hydrates sont données dans le tableau ci-après :

| Molécule | Structure | Petite cavité | | Grande cavité | |
|---|---|---|---|---|---|
| | | $A \times 10^3$ | B | $A \times 10^3$ | B |
| | | (K/atm) | (K) | (K/atm) | (K) |
| $N_2$ | I | 1,617 | 2905 | 6,078 | 2431 |
| | II | 0,1742 | 3082 | 18,00 | 1728 |
| $CO_2$ | I | 0,2474 | 3410 | 42,46 | 2813 |
| | II | 0,0845 | 3615 | 851,0 | 2025 |
| $H_2S$ | I | 0,0250 | 4568 | 16,34 | 3737 |
| | II | 0,0298 | 4878 | 87,2 | 2633 |
| $C_1$ | I | 0,7228 | 3187 | 23,35 | 2653 |
| | II | 0,2207 | 3453 | 100,0 | 1916 |
| $C_2$ | I | 0,0 | 0,0 | 3,039 | 3861 |
| | II | 0,0 | 0,0 | 240,0 | 2967 |
| $C_3$ | II | 0,0 | 0,0 | 5,455 | 4638 |
| $iC_4$ | II | 0,0 | 0,0 | 189,3 | 3800 |
| $nC_4$ | II | 0,0 | 0,0 | 30,51 | 3699 |

Calcul des fugacités :

Les fugacités des différents constituants présents dans le mélange, formateurs d'hydrates ou non, sont calculées à l'aide de l'équation d'état de Soave-Redlich-Kwong déjà citée. Un tel calcul se fait par l'appel au module de flash à pression et température fixées intégré dans TACITE.

Activité de l'eau en phase aqueuse :

Le module de Munck néglige la solubilité dans la phase aqueuse des hydrocarbures, de l'azote et du sulfure d'hydrogène. En revanche, la solubilité du dioxyde de carbone dans l'eau ne peut être négligée et est calculée à l'aide de la constante de Henry du $CO_2$ dans l'eau :

$$x_{CO_2} = \frac{f_{CO_2}}{H_{CO_2}}, \qquad\qquad (11)$$

où $x_{CO_2}$ est la fraction molaire de $CO_2$ dans la phase aqueuse, $f_{CO_2}$ la fugacité du $CO_2$ et $H_{CO_2}$ la constante de Henry du $CO_2$ dans l'eau donnée par :

$$\ln H_{CO_2} = H_A + \frac{H_B}{T} + H_C \ln T + H_D T. \qquad\qquad (12)$$

Les valeurs des coefficients utilisés pour le calcul de la constante de Henry du $CO_2$ figurent dans le tableau ci-après.

| $H_A$ (atm) | $H_B$ (atm.K) | $H_C$ (atm/lnK) | $H_D$ (atm.K$^{-1}$) |
|---|---|---|---|
| 160,27 | -8764,5 | -21,726 | 1,1055.10$^{-4}$ |

Constantes de référence :

La différence de potentiel chimique entre l'eau liquide pure et l'eau dans la phase hydrate vide (premier membre de l'équation 9), peut s'écrire, moyennant quelques approximations :

$$\frac{\mu_{eau}^{hydrate\text{-}vide}(P,T) - \mu_{eau}^{0}(P,T)}{RT} = \frac{\Delta\mu_0}{RT_0} - \int_{T_0}^{T}\frac{\Delta H_0 + \Delta C_p(T - T_0)}{RT^2}dT + \int_{P_0}^{P}\frac{\Delta V}{R\overline{T}}dP. \qquad (13)$$

Dans cette équation, $\Delta\mu_0$ représente la différence de potentiel chimique de l'eau dans le réseau hydrate vide et dans l'eau liquide à $T_0$ = 273,15 K. $\Delta H_0$ est la différence d'enthalpie correspondante, $\Delta C_p$ la différence de capacité calorifique et $\Delta V$ la différence de volume. La pression $P_0$ est la pression de vapeur à $T_0$ ; étant donné que $P_0$ est très petit comparé à $P$, on utilise $P_0$ = 0.
La dépendance en température du terme $PV$ est moyennée par :

$$\overline{T} = (T + 273{,}15)/2. \qquad\qquad (14)$$

$\Delta V$ et $\Delta H_0$ sont tous deux considérés comme étant indépendants de la pression. $\Delta V$ est considéré aussi comme indépendant de la température, alors que la dépendance en température du terme enthalpique est prise en compte par le biais d'une différence de capacité calorifique constante $\Delta C_p$. Les différentes constantes figurant dans l'équation (13) sont présentées dans le tableau suivant :

| Propriétés | Unités | Structure I | Structure II |
|---|---|---|---|
| $\Delta\mu_0$ | J.mol$^{-1}$ | 1264 | 883 |
| $\Delta H_0$ | J.mol$^{-1}$ | -4858 | -5201 |
| $\Delta V$ | cm$^3$.mol$^{-1}$ | 1151 | 808 |
| $\Delta C_\pi$ | J.mol$^{-1}$ .K$^{-1}$ | 39,16 | 39,16 |

La combinaison des équations (9) et (13) donne l'équation finale (15) :

EP 1 237 000 B1

$$\frac{\Delta\mu_0}{RT_0} - \int_{T_0}^{T} \frac{\Delta H_0 + \Delta C_P(T-T_0)}{RT^2}dT + \int_{P_0}^{P} \frac{\Delta V}{\overline{RT}}dP = \ln(\gamma x) - \sum_i v_i \ln\left(1 - \sum_k \theta_{ik}\right). \quad (15)$$

Dans cette équation les inconnues sont la pression et la température. Nous avons arbitrairement choisi de fixer la pression et de calculer la température d'équilibre des hydrates correspondante. La recherche de cette température, c'est-à-dire la résolution de l'équation (15), s'effectue de manière itérative par une méthode de Newton-Raphson partant d'une température initiale arbitrairement fixée à 0˚C. Nous avons vérifié que la valeur de cette initialisation n'influe pas sur le résultat obtenu.

Validation du module

Nous avons validé le programme sur un ensemble de 700 points d'équilibre expérimentaux issus de 36 références bibliographiques différentes. Les résultats sont présentés sous forme d'histogrammes où figurent en abscisse, l'écart entre la température de dissociation calculée et celle mesurée, et en ordonnée, le pourcentage de points pour lesquels ces écarts de température sont compris entre les limites indiquées en abscisse. Les points expérimentaux utilisés pour réaliser ces histogrammes correspondent à différents couples pression - température.

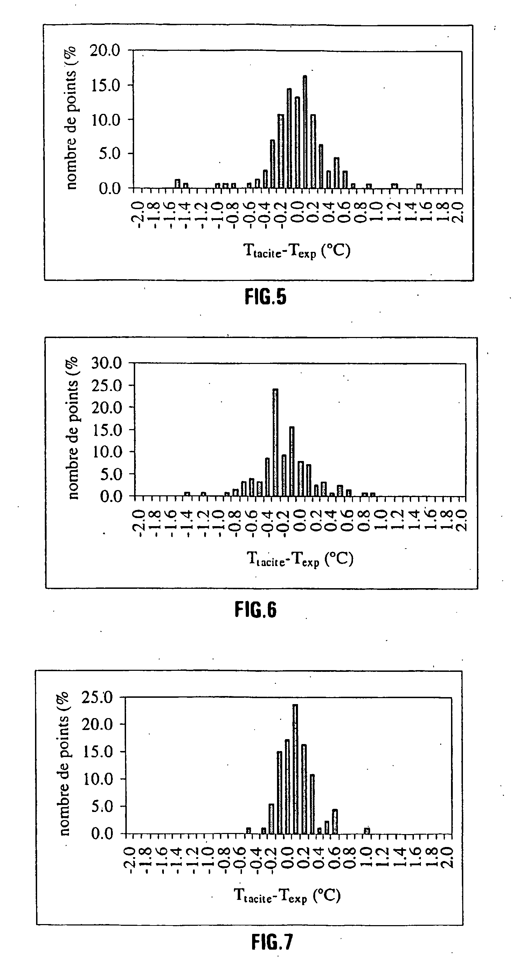

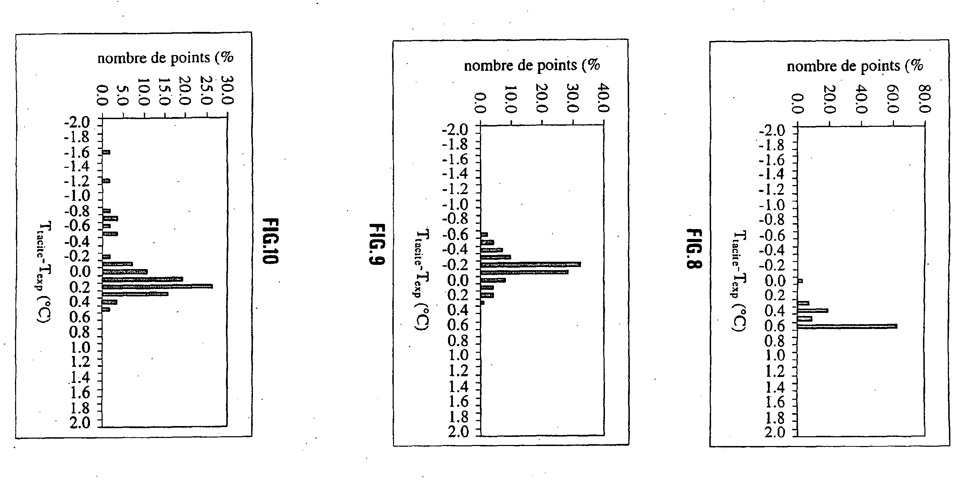

Sur les figures 5 à 11, nous comparons ainsi les résultats du calcul avec les données expérimentales dans le cas de divers corps purs : méthane, éthane, propane, isobutane, dioxyde de carbone, azote et sulfure d'hydrogène.

Nous constatons que sur l'ensemble des corps purs étudiés, le module utilisé donne d'excellentes prédictions des conditions d'équilibre des hydrates. Pour 99% des points, la température est prédite à $\pm 1$˚C. Dans tous les cas, l'écart maximal entre les températures de dissociation calculées et mesurées ne dépasse pas 1,6˚C.

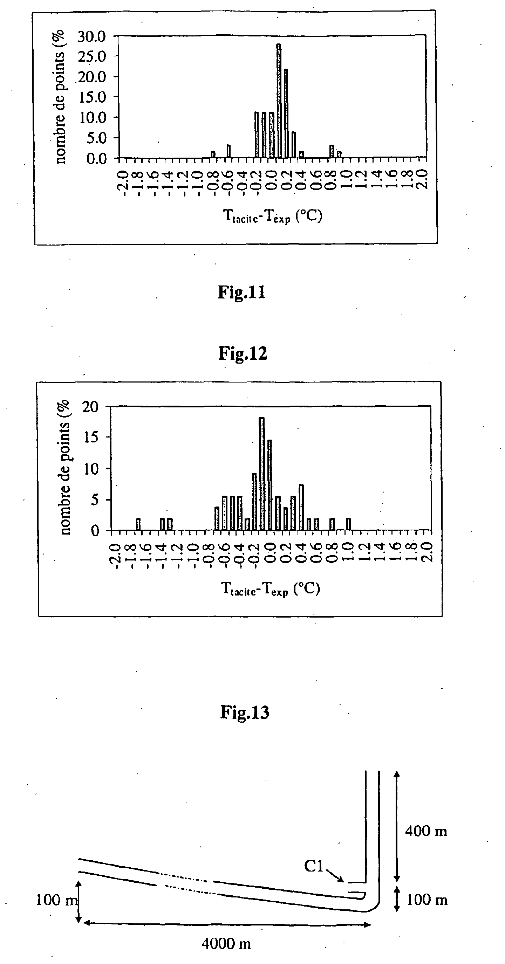

L'histogramme concernant les données de mélanges d'hydrocarbures, gaz naturels ou huiles, est présenté sur la Fig.12. Les données expérimentales utilisées pour calculer cet histogramme proviennent de huit mélanges différents, six de type gaz naturels et deux huiles. On observe à nouveau une très bonne description du comportement de ces systèmes par le module de prédiction utilisé.

Simulation globale avec ou sans calcul de risque d'apparition d'hydrates

L'impact se situe au niveau du regroupement des composants de base (lumping). Dans le cas standard, on cherche à en optimiser le nombre de façon à réduire le temps de calcul tout en gardant une bonne représentation des propriétés d'équilibre et des propriétés des phases. Dans le cas où l'on cherche à prédire les hydrates, il faut pouvoir suivre la composition des composants appelés formateurs d'hydrates et donc, ces composants ne doivent pas être regroupés avec d'autres.

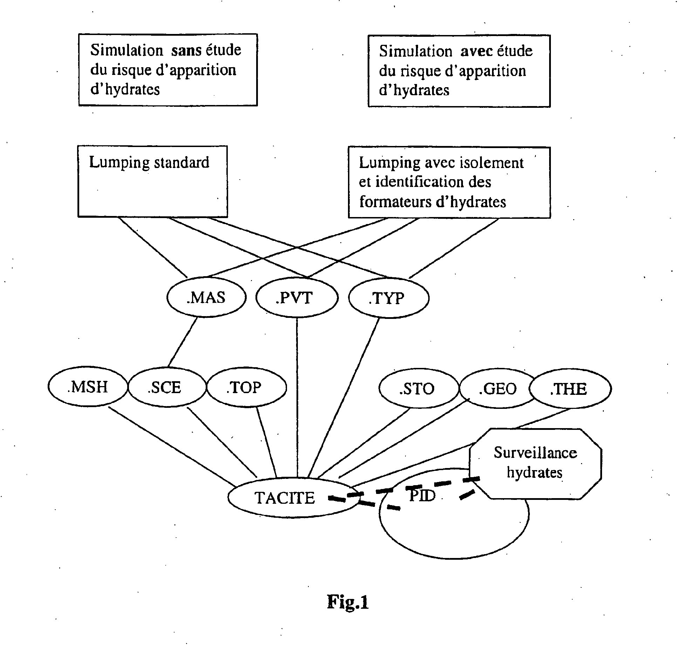

Outre les données se situant dans les fichiers de type « .PVT », « .MAS » et « .TYP » décrits précisément ci-après, les différentes données d'entrée de TACITE sont regroupées dans 6 fichiers (cf. Fig. 1):

-  « .SCE » : description du scénario, c'est à dire de l'évolution dans le temps de la valeur des différentes conditions aux limites (débit d'entrée, pression de sortie, ouverture de vanne,...);

-  « .TOP » : description de la topographie de la conduite (élévation de la conduite en fonction de la longueur) ;

-  « .GEO»: description de la géométrie de la conduite (diamètre, longueur, porosité) ;

-  « .THE » : description des isolations thermiques de la conduite ;

-  « .MSH » : description du maillage ;

-  « .STO » : description du type de fichier de résultats et de la fréquence de stockage des résultats.

**Fichier «.PVT»**

Le fichier «.PVT» contient autant de lignes que de données thermodynamiques du fluide à indiquer, respectivement :

-  un entier qui définit le type d'équation d'état utilisée : **3** pour Peng-Robinson ou **4** pour Soave-Redlich-Kwong.

- un entier qui définit le nombre de phases ; il est mis à **1** et c'est le flash qui détermine leur nombre.
- le nombre de composants.
- Boucle sur les composants avec indication, pour chacun d'eux, de leur :

- masse molaire [kg/mol].
- température critique [K].
- pression critique [Pa].
- volume critique [$m^3$/mol].
- facteur acentrique [-].
- parachor [$((N/m)^{1/4}m^3/mol)$].
- volume molaire standard (inutilisé).
- facteur de correction volumique [$m^3$/mol].
- coefficients d'enthalpie idéale (tableau de 7 coefficients décrits ci-dessous).
- Fin de la boucle.
- la matrice des coefficients d'interaction binaire [-].
- la température de calage 1 [K], Température de calage 2 [K].
- la viscosité cinématique à la température 1 [$m^2$/s], Viscosité cinématique à la température 2 [$m^2$/s].

### Fichier «.MAS»

Ce fichier comporte également un certain nombre de lignes pour indiquer :

- le nombre de composants initiaux.
- la liste du numéro du groupe dans lequel le composant initial correspondant a été mis. L'ordre est celui dans lequel se trouvaient les composants dans le fichier «.INP ».
- le nombre de pseudo-composants.
- les différentes fractions massiques des composants, ordonnées comme dans le fichier «.PVT».

### Fichier «.TYP»

Ce fichier contient autant de lignes que de pseudo-composants définis dans le fichier «.PVT» et les informations sur les composants tiennent compte de l'ordre dans lequel les composants sont rangés dans le fichier «.PVT».
La i$^{ème}$ ligne concerne l'identificateur de la nature du i$^{ème}$ composant.
La convention suivante est retenue :

Si le composant est ni l'eau, ni un formateur d'hydrates, son type est mis à **0.**
Si le composant est le sulfure d'hydrogène, son type est mis **à 1.**
Si le composant est l'azote, son type est mis à **2.**
Si le composant est le dioxyde de carbone, son type est mis **3.**
Si le composant est le méthane, son type est mis à **4.**
Si le composant est l'éthane, son type est mis à **5.**
Si le composant est le propane, son type est mis à **6.**
Si le composant est l'iso-butane, son type est mis à **7.**
Si le composant est le n-butane, son type est mis à **8.**
Si le composant est l'eau, son type est mis à **9.**

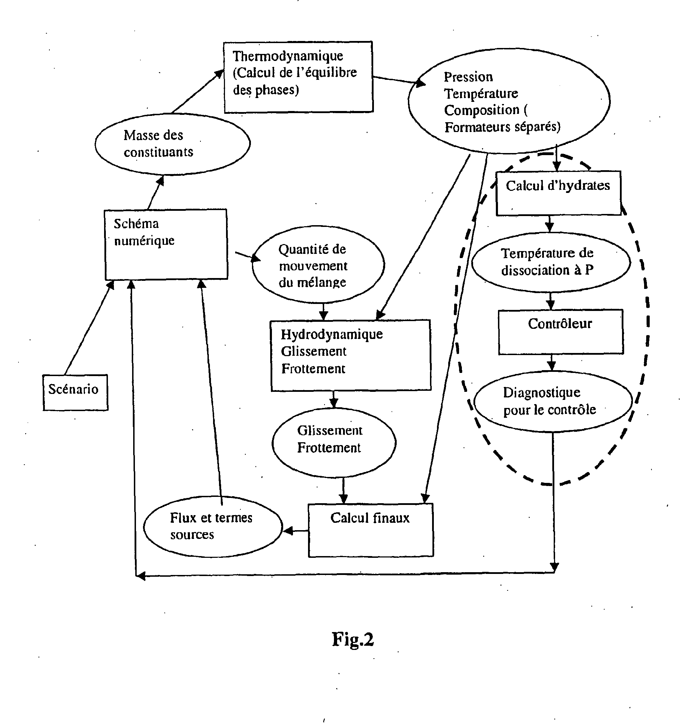

Le code de simulation TACITE utilise une méthode de résolution numérique de type à volumes finis pour simuler les écoulements dans les installations de production, les conduites étant discrétisées par exemple selon la méthode de maillage décrite dans la demande de brevet FR EN 00/08200 du demandeur.
A tout instant, en tout point du maillage, les calculs de modélisation sont conduits selon l'organigramme de la Fig.2.

✓ Le schéma numérique est alimenté par la donnée des termes de flux et les termes source, la modification des conditions aux limites (scénario).

✓ Le schéma numérique fournit les masses des constituants, la quantité de mouvement du mélange, l'énergie interne du mélange (non-isotherme).

✓ La connaissance des masses et de l'énergie fournit, grâce au flash thermodynamique, la pression, la température et la composition massique des phases à l'équilibre.

✓ Connaissant la pression et la composition massique, il est possible, si l'utilisateur le souhaite (voir partie entourée d'un trait en pointillés), de :

- calculer précisément grâce au module de prédiction d'hydrates, la température Td de dissociation des hydrates (température au-dessous de laquelle si des hydrates se sont formés, ils ne peuvent se dissocier) ;

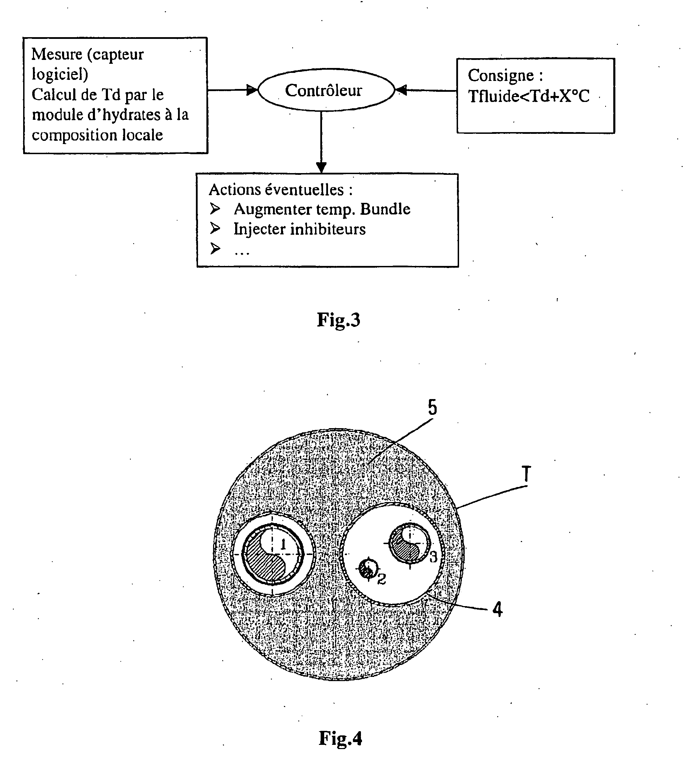

- d'utiliser un contrôleur C de type PID (Fig.3) pour comparer la température calculée du fluide (Tfluide) à cette température Td de dissociation ; et

- d'agir si cette température de fluide est inférieure à la température de dissociation majorée d'un petit écart (pour anticiper le problème éventuel) sur l'un des moyens de contrôle prévu .

✓ Indépendamment de ce contrôle éventuel, le calcul continue, la connaissance de l'équilibre du mélange et des propriétés des phases, associée à la connaissance de la quantité de mouvement du mélange permet, grâce au module hydrodynamique, de fournir le glissement entre les phases et les frottements pariétaux et interfaciaux.

✓ Il est alors possible de calculer les termes sources et les flux pour le prochain calcul.

## Contrôle en ligne

Les opérations de lutte contre la formation d'hydrates sont généralement très coûteuses. La modélisation fine permise par la méthode selon l'invention, permet à l'exploitant de connaître à tout instant les conditions thermodynamiques régnant tout le long du pipeline et donc de déterminer les endroits où les hydrates sont susceptibles de se former. Il est en mesure quand le besoin s'en fait sentir et seulement à ces moments-là, de mettre en oeuvre des actions pour combattre les dépôts d'hydrates. Les nécessités d'intervention sont plus particulièrement critiques en cas d'arrêt momentané de la production ou quand on procède à des injections de gaz dans le mélange en circulation (aux emplacements de « gas lift ») par exemple.

Un premier mode d'action consiste à réchauffer les fluides en circulation de manière à les amener au-dessus de la température de dissociation d'hydrates que l'on a prédite, au moins aux endroits de la conduite où le risque est apparu. Les conduites pétrolières pour la production marine par grands fonds sont généralement incluses avec d'autres lignes dans une conduite métallique T revêtue extérieurement d'une couche isolante. Cet ensemble de lignes (désigné par « bundies » par les gens de l'art) est réparti souvent en plusieurs faisceaux tels que F1, F2 (cf. Fig.4). Chaque faisceau comporte généralement une ou plusieurs lignes de transport 1, 2, 3 pour fluides monophasiques ou polyphasiques qui peuvent être rassemblées dans un tube commun 4 éventuellement recouvert lui-même extérieurement d'une couche isolante. L'intérieur de chaque tube 4 est rempli le plus souvent d'un fluide monophasique . L'espace intérieur de la conduite T, entre les différents faisceaux F1, F2, est rempli par exemple d'une mousse thermiquement isolante 5. L'une des lignes de transport 2, 3 peut être utilisée par exemple pour faire circuler si besoin est, un fluide de réchauffage.

Pour modéliser les transferts thermiques entre les conduites pétrolières de l'ensemble de lignes et leur environnement (autres lignes, milieu extérieur) en tenant compte des moyens d'isolation thermique intercalés, on peut se reporter par exemple à la publication suivante :

- Duret, E. et al, "Pipeline bundles model implemented into a multiphase flow model", SPE 62948, SPE Annual Technical Conference and Exhibition, Dallas, Texas, 1-4 October (2000).

Un deuxième mode d'action consiste à injecter dans les fluides des additifs inhibiteurs de type connu s'opposant à la formation d'hydrates.

Il s'agit d'un système de contrôle basé, non pas sur une mesure fournie par un capteur réel, mais sur une mesure fournie par un capteur dit « logiciel ».

## Intérêt d'un code compositionnel pour un calcul d'hydrates

L'utilisation d'un code compositionnel tel que le code TACITE permet en tout point du pipeline, un calcul exact, à la pression calculée du fluide et pour la composition locale calculée en cours de simulation, de la température de dissociation des hydrates, et par conséquent une bien meilleure simulation des risques d'apparition d'hydrates que des codes où la composition est supposée fixe, correspondant à celle que l'on injecte à l'entrée de la conduite. On a pu vérifier par exemple que l'injection de gaz au pied d'un « riser » (technique bien connue des gens de l'art), modifiait les conditions thermodynamiques locales d'un mélange donné de fluides pétroliers, au point que l'on pouvait prédire la formation d'hydrates, alors qu'en supposant une composition fixe du mélange, ce risque n'était aucunement détecté. Nous illustrons ce point avec la simulation effectuée d'une injection de gaz au pied d'un riser se raccordant à un pipeline tel que celui schématisé sur la Fig.13, alimenté par un mélange dont la composition est donnée dans le tableau ci-dessous.

| Components | Composition (% mol.) |
|---|---|
| $C_1$ | 10.53 |
| $C_2$ | 2.64 |
| $C_3$ | 2.63 |
| $IC_4$ | 2.63 |
| $NC_4$ | 2.63 |
| $IC_5$ | 2.63 |
| $NC_5$ | 2.63 |
| $NC_{10}$ | 13.16 |
| $NC_{20}$ | 7.90 |
| $H_2O$ | 52.62 |

La simulation est effectuée en considérant que le mélange initial a été modifié par une injection en bas du riser, de méthane favorisant la formationd'hydrates.

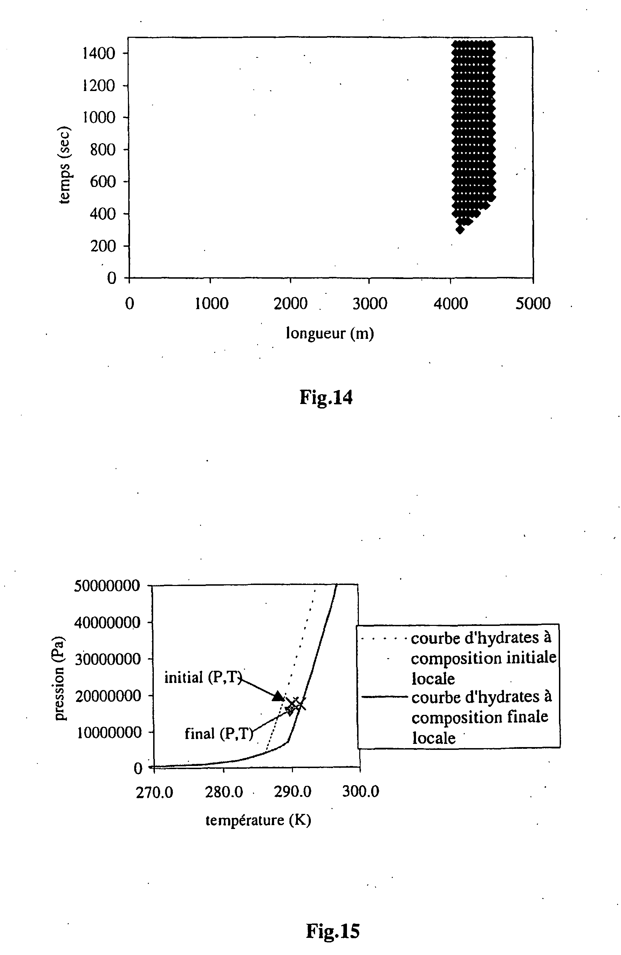

Dans un diagramme longueur de conduite-temps, le domaine d'apparition d'hydrates que l'on obtient est schématisé à la Fig.14.

On a calculé et représenté sur le diagramme bidimensionnel pression-temps de la Fig.15, deux courbes locales de formation d'hydrates, l'une (en pointillés) en considérant que la composition n'a pas été altérée par l'injection de méthane, comme le ferait un code de simulation non compositionnel, l'autre (en traits pleins) en tenant compte de la modification de composition intervenue.

On vérifie aisément sur la Fig.15, que le point qui y est figuré représentant le couple (pression, température) à l'état final serait considéré à tort comme sans risque puisqu'à droite de la courbe d'hydrates calculée à la composition initiale. Le domaine de risque d'apparition d'hydrates, présenté sur la Fig.14, serait également modifié en raison des variations de composition.

**Revendications**

1.   Méthode pour détecter en continu les conditions thermodynamiques de formation d'hydrates en tout point d'une conduite (1) où circule un mélange polyphasique de fluides pétroliers, dans laquelle on utilise un module hydrodynamique mécanistique et un module thermodynamique compositionnel intégré pour définir les propriétés des phases et l'on applique des équations de conservation de la masse et de la quantité de mouvement, et de transfert d'énergie au sein du mélange, en considérant que le mélange de fluides est sensiblement à l'équilibre à chaque instant, que la composition du mélange polyphasique est variable tout le long de la conduite et que la masse de chaque constituant du mélange est définie globalement par une équation de conservation de la masse sans considération de son état de phase, et on regroupe les fluides pétroliers en un nombre limité de pseudo-composants, **caractérisée en ce que** l'on détecte les conditions thermodynamiques de formation d'hydrates :

   - en opérant un regroupement des fluides pétroliers en pseudo-composants dans lequel les composants formateurs d'hydrates sont isolés, et en définissant pour chaque pseudo-composant des données comportant une fraction massique et des grandeurs physiques caractéristiques du pseudo-composant ; et
   - en appliquant aux dits modules, les données définies pour lesdits pseudo-composants, de manière à déterminer en tout point la température (Td) de dissociation des hydrates.

2.   Méthode selon la revendication 1, dans laquelle on contrôle en continu la formation d'hydrates en tout point de la conduite en appliquant les étapes suivantes :

a) on utilise un organe de contrôle (C) pour comparer la température des fluides pétroliers à ladite température (Td) de dissociation des hydrates ; et

b) on applique sous le contrôle de cet organe de contrôle (C), des mesures destinées à combattre la formation des hydrates.

**3.** Méthode selon la revendication 2, **caractérisée en ce que** l'on utilise des moyens de chauffage associés à la conduite (1) pour élever la température des fluides pétroliers au-dessus de la température de dissociation.

**4.** Méthode selon la revendication 3, **caractérisée en ce que**, la conduite (1) étant incluse avec au moins un deuxième conduit (2, 3) dans un tube (T) isolé du milieu extérieur, on utilise le deuxième conduit (2, 3) pour y faire circuler un fluide chaud.

**5.** Méthode selon la revendication 2, **caractérisée en ce que**, sur commande de l'organe de contrôle (C), on injecte des inhibiteurs d'hydrates dans la conduite (1).

**Claims**

**1.** A method intended for continuous detection, at any point of a pipe carrying a multiphase mixture of petroleum fluids, of thermodynamic hydrate formation conditions using a mechanistic hydrodynamic module and an integrated compositional thermodynamic module to define the phase properties, and mass conservation and momentum conservation equations, as well as equations of energy transfer in the mixture are applied, considering that the mixture of fluids is substantially continuously at equilibrium, that the composition of the multiphase mixture is variable all along the pipe and that the mass of each constituent of the mixture is globally defined by a mass conservation equation regardless of its phase state, and the petroleum fluids are lumped together into a limited number of pseudo-components, **characterized in that** the thermodynamic hydrate formation conditions are detected :

- by carrying out a particular lumping of the petroleum fluids into pseudo-components so as to isolate the hydrate forming components, with definition for each one of a mass fraction and of a certain number of characteristic physical quantities, and
- by applying to said modules data relative to these particular fractions so as to determine at any point the hydrate dissociation temperature (Td).

**2.** A method intended for continuous control of hydrate formation at any point of a pipe carrying a multiphase mixture of petroleum fluids, using a mechanistic hydrodynamic module and an integrated compositional thermodynamic module to define the phase properties, and mass conservation and momentum conservation equations, as well as equations of energy transfer in the mixture are applied, considering that the mixture of fluids is substantially continuously at equilibrium, that the composition of the multiphase mixture is variable all along the pipe and that the mass of each constituent of the mixture is globally defined by a mass conservation equation regardless of its phase state, and the petroleum fluids are lumped together into a limited number of pseudo-components, **characterized in that** :

a) the thermodynamic hydrate formation conditions are detected by :

- carrying out a particular lumping of the petroleum fluids into pseudo-components so as to isolate the hydrate forming components, with definition for each one of a mass fraction and of a certain number of characteristic physical quantities, and
- by applying to said modules data relative to these particular fractions so as to determine the hydrate dissociation temperature (Td) ;

b) a control device (C) is used to compare the temperature of the petroleum fluids with this dissociation temperature (Td) ; and

c) measures intended to fight hydrate formation are applied under the control of this control device (C).

**3.** A method as claimed in claim 2, **characterized in that** heating means associated with pipe (1) are used to raise the temperature of the petroleum fluids above the dissociation temperature.

**4.** A method as claimed in claim 3, **characterized in that**, pipe (1) being included with at least a second pipe (2, 3) in a tube (T) isolated from the outside medium, second pipe (2, 3) is used for circulation of a warm fluid.

5. A method as claimed in claim 2, **characterized in that** hydrate inhibitors are injected into pipe (1) under the control of control device (C).

**Patentansprüche**

1. Verfahren zum kontinuierlichen Erfassen der thermodynamischen Bedingungen der Hydratbildung an jedem Punkt einer Leitung (1), in der ein mehrphasiges Gemisch aus Erdölfluiden zirkuliert, wobei ein mechanistisches hydrodynamisches Modul und ein integriertes thermodynamisches Modul für die Zusammensetzung verwendet wird, um die Eigenschaften der Phasen zu definieren, und Masseerhaltungs- und Impulserhaltungsgleichungen sowie Gleichungen der Energieübertragung innerhalb des Gemischs angewendet werden, wobei berücksichtigt wird, dass das Fluidgemisch im Wesentlichen zu jedem Zeitpunkt im Gleichgewicht ist, dass die Zusammensetzung des mehrphasigen Gemischs über die gesamte Leitung variabel ist und dass die Masse jedes Bestandteils des Gemischs insgesamt, ohne Berücksichtigung seines Phasenzustands, durch eine Masseerhaltungsgleichung definiert wird, und die Erdölfluide in eine begrenzte Anzahl von Pseudobestandteilen umgruppiert werden, **dadurch gekennzeichnet, dass** die thermodynamischen Bedingungen der Hydratbildung erfasst werden:

   - indem eine Umgruppierung der Erdölfluide in Pseudobestandteile vorgenommen wird, wobei die Bestandteile, die Hydratbilder sind, isoliert werden, und indem für jeden Pseudobestandteil Daten definiert werden, die eine Massefraktion und physikalische Größen umfassen, die für den Pseudobestandteil kennzeichnend sind; und
   - indem auf die Module die für die Pseudobestandteile definierten Daten angewendet werden, um an jedem Punkt die Temperatur der Hydratdissoziation (Td) zu bestimmen.

2. Verfahren nach Anspruch 1, wobei an jedem Punkt der Leitung die Hydratbildung kontinuierlich kontrolliert wird, indem die folgenden Schritte angewendet werden:

   a) Es wird eine Kontrollvorrichtung (C) verwendet, um die Temperatur der Erdölfluide mit der Hydratdissoziationstemperatur (Td) zu vergleichen; und
   b) unter der Kontrolle dieser Kontrollvorrichtung (C) werden Maßnahmen angewendet, die dazu bestimmt sind, der Hydratbildung entgegenzuwirken.

3. Verfahren nach Anspruch 2, **dadurch gekennzeichnet, dass** Mittel zum Erwärmen verwendet werden, die mit der Leitung (1) assoziiert sind, um die Temperatur der Erdölfluide über die Dissoziationstemperatur zu erhöhen.

4. Verfahren nach Anspruch 3, **dadurch gekennzeichnet, dass**, wobei Leitung (1) zusammen mit mindestens einer zweiten Leitung (2, 3) in einem Rohr (T) enthalten ist, das vom umgebenden Medium isoliert ist, die zweite Leitung (2, 3) verwendet wird, um dort ein warmes Fluid zirkulieren zu lassen.

5. Verfahren nach Anspruch 2, **dadurch gekennzeichnet, dass**, gesteuert durch die Kontrollvorrichtung (C), Hydratinhibitoren in die Leitung (1) injiziert werden.

Fig.1

**Fig.2**

```
┌────────────────────────┐        ┌──────────────────┐
│ Mesure (capteur        │        │ Consigne :       │
│ logiciel)              │──▶ Contrôleur ◀──│ Tfluide<Td+X°C │
│ Calcul de Td par le    │        │                  │
│ module d'hydrates à la │        └──────────────────┘
│ composition locale     │
└────────────────────────┘
              │
              ▼
┌────────────────────────────┐
│ Actions éventuelles :      │
│ ➢ Augmenter temp. Bundle   │
│ ➢ Injecter inhibiteurs     │
│ ➢ …                        │
└────────────────────────────┘
```

**Fig.3**

**Fig.4**

19

FIG.5

FIG.6

FIG.7

**FIG.8**

**FIG.9**

**FIG.10**

**Fig.11**

**Fig.12**

**Fig.13**

**Fig.14**

initial (P,T)

final (P,T)

· · · · courbe d'hydrates à
composition initiale
locale
—— courbe d'hydrates à
composition finale
locale

**Fig.15**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2753535
- FR 2806803
- US 5550761 A
- FR 2756044
- US 6028992 A
- FR 2756045
- US 5960187 A
- FR 0008200
- FR 0009889

**Littérature non-brevet citée dans la description**

- **D.-Y. Peng D.Y. et al.** A new two-constant equation of state. *Ind. Eng. Chem. Fund.,* 1976, vol. 15, 59
- **Soave G.** Equilibrium constants from a modified Redlich-Kwong equation of state. *Chem. Eng. Sci.,* 1972, vol. 27, 1197
- **Péneloux A. et al.** A consistent correction for Redlich-Kwong-Soave volumes. *Fluid Phase Equilibria,* 1982, vol. 8, 7
- **Munck, J. et al.** Computations of the formation of gas hydrates. *Chem. Eng. Sci,* 1988, vol. 43, 2661
- **Duret, E. et al.** Pipeline bundles model implemented into a multiphase flow mode. *SPE 62948, SPE Annual Technical Conference and Exhibition,* 01 Octobre 2000